# EUROPÄISCHE PATENTSCHRIFT

(11) **EP 1 763 534 B1**
(45) Veröffentlichungstag und Bekanntmachung des Hinweises auf die Patenterteilung: **24.02.2010**
(21) Anmeldenummer: 05768705.5
(22) Anmeldetag: 04.07.2005
(51) Int. Cl.: C07J 63/00, A61K 31/56, A61P 5/32

(54) **NEUE 2-SUBSTITUIERTE D-HOMO ESTRA-1,3,5(10)-TRIENE ALS INHIBITOREN DER 17ß-HYDROXYSTEROIDDEHYDROGENASE TYP 1**
NOVEL 2-SUBSTITUTED D-HOMO-ESTRA-1,3,5(10)-TRIENES AS INHIBITORS OF 17ß-HYDROXYSTEROID DEHYDROGENASE TYPE 1
NOUVEAUX D-HOMO-ESTRA-1,3,5(10)-TRIENES 2-SUBSTITUES SERVANT D'INHIBITEURS DE LA 17ß-HYDROXYSTEROIDE-DESHYDROGENASE DE TYPE 1

(30) Priorität: 02.07.2004 DE 102004032673
(43) Veröffentlichungstag der Anmeldung: 21.03.2007
(73) Patentinhaber: Bayer Schering Pharma Aktiengesellschaft, 13353 Berlin (DE)
(72) Erfinder: GEGE, Christian, 89584 Ehingen (DE); REGENHARDT, Wilko, 81243 München (DE); PETERS, Olaf, 99891 Tabarz (DE); HILLISCH, Alexander, 42553 Velbert (DE); ADAMSKI, Jerzy, 80809 München (DE); MÖLLER, Gabriele, 80992 München (DE); DELUCA, Dominga, 37060 Buttapietra (VR) (IT); ELGER, Walter, 14195 Berlin (DE); SCHNEIDER, Birgitt, 07745 Jena (DE)
(86) Internationale Anmeldenummer: PCT/EP2005/007314
(87) Internationale Veröffentlichungsnummer: WO 2006/003012

(56) Entgegenhaltungen:
- WO-A-03/017973
- WO-A-2004/074309
- US-A- 3 005 835
- US-B1- 6 541 463
- EGOROVA, V. V. ET AL: "Structure and reactivity of steroids. VI. Long range effects in estra--1,3,5-(10)-triene compounds" TETRAHEDRON , 29(2), 301-7 CODEN: TETRAB; ISSN: 0040-4020, 1973, XP002350863

## Beschreibung

Die vorliegende Erfindung betrifft neue 2-substituierte D-Homo-Estra-1,3,5(10)-triene, deren Herstellung und Verwendung als Arzneimittel zur Behandlung estrogen-abhängiger Erkrankungen, die sich durch Hemmung der 17β-Hydroxysteroiddehydrogenase Typ 1 beeinflussen lassen sowie pharmazeutische Zusammensetzungen, die diese Verbindungen enthalten.

WO-A-03/017973 beschreibt eine 16-substituierte Estradiol-verbindung zur Hemmung von 17β-Hydroxysteroiddehydrogenase -Typ-1.

Sexualhormone kontrollieren die Proliferation und Funktion von steroidsensitivem normalen sowie malignen Gewebe [E. E. Baulieu, Hormones, a complex communication network. In Hormones, eds. E. E. Baulieu and P.A. Kelly, Herman Publisher Paris and Chapman and Hall New York, 1990, pp. 147-149; D.D. Thomas, Cancer 53 (1984) 595-601].

Estradiol ist das aktivste weibliche Sexualhormon, welches außer den bekannten Effekten auf das Reproduktionssystem weitere Funktionen beim Knochen- und Lipidmetabolismus, im kardiovaskulären System sowie regulatorische Effekte im zentralen Nervensystem ausübt. Es wird bei prämenopausalen Frauen primär in den Ovarien produziert. Ein weiterer großer Teil der aktiven Estrogene wird im peripheren Gewebe aus inaktiven Steroidprecursoren gebildet, die beim Menschen in den Nebennieren in großen Mengen in das Blut ausgeschüttet werden.
Nach der Menopause sinkt der Estradiolspiegel im Blut auf ca. 1/10 des Gehalts von prämenopausalen Frauen [T.Thorsten, M. Tangen, K. F. Stoa, Eur. J. Cancer Clin. Oncol. 18 (1982) 333-337; A. A. van Landeghem et al., Cancer Res. 45 (1985) 2900-2906]. Ab diesem Zeitpunkt werden Estrogene hauptsächlich über die Biosynthese im peripheren Gewebe zur Verfügung gestellt [F. Labrie, Intracrinology. Mol. Cell. Endocrinol. 78(1991) C113-C118].

Estrogene werden über das Blut vom Tumorgewebe aufgenommen und stimulieren dessen Wachstum.
Die Konzentration des intratumoralen Estradiols bleibt jedoch auch nach der Menopause auf hohem Niveau erhalten, vergleichbar dem bei prämenopausalen Frauen [A. A. van Landeghem et al., Cancer Res. 45 (1985) 2900-2906]. Die hohe Estradiolkonzentration im Tumorgewebe bei postmenopausalen Frauen wird durch Biosynthese von Estrogenen im Tumorgewebe verursacht.

Estradiol (E2) wird im Brustkrebsgewebe entweder über den Aromatase- oder den Sulfataseweg gebildet [Y. J. Abul-Hajj, R. Iverson, D. T. Kiang, Steroids 33 (1979) 205-222; A. Lipton et al., Cancer 59 (1987), 779-782; E. Perel et al., J. Steroid Biochem. 29 (1988) 393-399]. Androstendion wird aus dem Blut vom Tumorgewebe aufgenommen, zu Estron (E1) aromatisiert und anschließend zu Estradiol (E2) reduziert (Aromataseweg). Beim Sulfataseweg wird Estronsulfat durch die Steroidsulfatase in E1 umgewandelt und wiederum zu E2 reduziert.

Der entscheidende letzte Schritt der Steroidsynthese wird von 17β-Hydroxysteroiddehydrogenasen (17β-HSD), zugehörig zur Familie der 17β-Hydroxysteroiddehydrogenasen/ 17-Ketosteroidreduktasen katalysiert. Diese Enzyme wandeln geringer aktive 17-Ketosteroide in deren aktive 17β-Hydroxysteroide um und umgekehrt. Sowohl Estrogene als auch Androgene zeigen die höchste Affinität zu den entsprechenden Rezeptoren in der 17β-Hydroxyform, d.h. die 17β-HSD-Enzyme steuern die biologische Aktivität der Sexualhormone [H. Peltoketo et al., J. Mol. Endocrinol. 23 (1999), 1-11; P. Vihko et al., Mol. Cell. Endocrinol. 171 (2001) 71-76].
Bestimmte extragonadale Gewebe wie Brust- und Prostatagewebe exprimieren reduktive 17-HSD's und wandeln so die im Blut zirkulierenden Vorstufen mit geringerer Aktivität in den Zielgeweben in die aktiveren Formen um [F. Labrie et al., Steroids 62 (1997) 148-158; H. Peltoketo et al., Horm. 55 (1999) 353-398].

Bis heute sind 11 verschiedene 17β-HSD's bekannt. Sie unterscheiden sich in ihrer Gewebeverteilung, der katalytischen Aktivität, in ihrer Substratspezifität, der subzellulären Lokalisation und durch den Regulationsmechanismus. Für eine große Anzahl der Hydroxysteroiddehydrogenasen konnte deren Beteiligung an der Pathogenese von Erkrankungen des Menschen gezeigt werden, bsplw. für Pseudohermaphroditismus [17β-HSD 3, W. M. Geissler et al., Nat. Genet. 7 (1994) 34-39], bifunktionales Enzymdefizit [17β-HSD 4, E. G. van Grunsven et al., Proc. Natl. Acad. Sci. USA 95 (1998) 2128-2133], polyzystische Nierenerkrankung [17β-HSD 8, M. M. Maxwell et al., J. Biol. Chem. 270 (1995) 25213-25219] und die Alzheimer-Erkrankung [17β-HSD 10, S. D. Yan et al., Nature 389 (1997) 689-695; X. Y. He et al., J. Biol. Chem. 274 (1999) 15014-15019].

Die humanen plazentalen 17β-Hydroxysteroiddehydrogenasen Typ 1 und Typ 2 gehören derselben Steroiddehydrogenase-Reduktase-Proteinfamilie (SDR) an. Sie unterscheiden sich voneinander unter anderem durch die Reaktionsrichtung, die von den Enzymen katalysiert wird.

17β-HSD 1 steuert vor allem die Reduktion von Estron zu Estradiol [T. Puranen et al., Endocrinology 138 (1997) 3532-3539] unter Beteiligung von NADPH als Co-Faktor [J. Z. Jin, S. X. Lin, Biochem. Biophys. Res. Commun. 259 (1999) 489-493].
In kultivierten Zellen unterstützt die HSD 1 teilweise die Reduktion von Androstendion und Androstandion. Es konnte aber eindeutig gezeigt werden, dass phenolische Substrate bevorzugt werden [M. Poutanen et al., Endocrinology 133 (1993) 2639-2644]. Im Vergleich mit 17β-HSD 1 katalysiert die 17β-HSD 2 dagegen die entgegengesetzte Reaktion, nämlich die Umwandlung von Estradiol zu Estron und von Androstendion und Dihydrotestosteron zu Androstandion [L. Wu et al., J. Biol. Chem. 268 (1993) 12964-12969] und agiert bevorzugt in Anwesenheit der nicht-phosphorylierten Form des Co-Faktors NAD [F. Labrie et al., Steroids 62 (1997) 148-158].

17β-HSD 1 und 2 werden in normalem Brustdrüsengewebe exprimiert [G. Söderqvist, J. Clin. Endocrinol. Metab. 83 (1998) 1190-1193; M. Miettinen, Breast Cancer Res, Treat. 57(1999) 175-182].
Im Gegensatz zu normalem Brustgewebe findet man in malignen Brustepithelzellen die reduktive Aktivität (durch 17β-HSD 1) gegenüber der oxidativen (durch 17β-HSD 2) erhöht [M. M. Miettinen et al., Biochem. J. 314 (1996) 839-845; V. Speirs, J. Steroid Biochem. Mol. Biol. 67 (1998) 267-274]. Es wurde beobachtet, dass Estradiol in malignen Brustzellen akkumuliert wird, was ebenfalls auf eine Aktivität der 17β-HSD 1 hinweist [A. Vermeulen et al., Eur. J. Cancer Clin. Oncol. 22 (1986) 515-525]. Außerdem wurde gefunden, dass in Anwesenheit von 17β-HSD 1 die Verabreichung von Estron in gleicher Weise zu einem Wachstum von Brustkrebszellen führt wie die Verabreichung von Estradiol allein. Im Gegensatz dazu bewirkt die Verabreichung von Estron allein ohne 17β-HSD 1 diesen Effekt nicht [M. M. Miettinen et al., Int. J. Cancer 68 (1996) 600-604].
Die Dominanz der 17β-HSD 1 in malignem Gewebe führt zu einem verstärkten Estrogen-abhängigen Wachstum und Fortschritt von Tumoren, während die oxidative 17β-HSD 2 normale Brustgewebezellen vor einem exzessiven Estradioleffekt schützen [P. Vihko et al. Mol. Cell. Endocrinol. 171 (2000) 71-76].
Im Falle der Endometriose spielt das Gleichgewicht zwischen der 17β-HSD 1 und 2 eine Rolle. 17β-HSD 1 wird in eutopischem Gewebe exprimiert, das Hormoninaktivierende Enzym 17β-HSD 2 fehlt jedoch völlig [S. E. Bulun et al. J. Mol. Endocrinol. 25 (2000) 35-42.
Auch bei Prostatacarcinomen ist 17β-HSD 2 erniedrigt [J. P. Elo et al., Endocrinol. Metab. 88 (2003) 705-712].

Unter den bisher entwickelten 17β-HSD 1-Inhibitoren unterscheidet man die irreversiblen von den reversiblen Inhibitoren. Die irreversiblen Inhibitoren enthalten eine reaktive funktionelle Gruppe, welche durch Bildung einer kovalenten Bindung mit einem Aminosäurerest des Enzyms dieses inaktiviert. Bekannte Vertreter der genannten Gruppe sind 16-Methylen-estradiole, Acetylen-substituiertes 16-Seco-estradiol [R. J. Auchus, D. F. Covey, Biochemistry 25 (1983) 7295-7300; J. L. Thomas et al., J. Biol. Chem. 258 (1983) 11500-11504; B. Tobias et al., J. Biol. Chem. 257 (1982) 2783-2786] oder auch 16α-Halogenalkyl-estradiole [K. M. Sam et al., Drug Des. Discov. 15 (1997) 157-180; M. R. Tremblay, D. Poirier, J. Steroid Biochem. Mol. Biol. 66 (1998) 179-191]. Zu den reversiblen Inhibitoren gehören 16,17-Pyrazol- oder 16,17-Isoxazol-estronderivate [F. Sweet et al. Biochem. Biophys. Res. Commun. 180 (1991), 1057-1063], Estradiolderivate mit einer langen 7α-Undecanamid- [C. Labrie et al. Cancer Res. 52 (1992), 610-615; S. J. Santner, R. J. Santen, J. Steroid Biochem. Mol. Biol. 45 (1993) 383-390] oder mit einer 6β-Thiaheptanamid-Seitenkette [D. Poirier, P. Dionne, S. Auger, J. Steroid Biochem. Mol. Biol. 64 (1998), 83-90].
Ein Spezialfall als 17β-HSD 1-Hemmer ist das 16-Oxoestron: es zählt bei neutralem pH-Wert 7.2 zu den reversiblen, unter basischen Bedingungen bei pH 8.5 zu den irreversiblen Inhibitoren [H. Inano, B. Tamaoki, Eur. J. Biochem. 129 (1983) 691-695].

Die bisher bekannten sowohl reversiblen als auch die irreversiblen Inhibitoren besitzen nur eine mäßige Aktivität als 17β-HSD 1 - Hemmer.

Kürzlich wurde durch Modelling-Untersuchungen der erste Hybrid-Inhibitor gefunden [M. Tremblay, D. Poirier et al., FASEB 13 (2002) 1829-1831]. Der Hybrid-Inhibitor, bei dem Estradiol mit Adenosin über eine Spacer aus 8 Methylengruppen an 16-Stellung verknüpft ist, hemmt die 17β-HSD 1 als bisher bester Inhibitor mit einem IC₅₀-Wert von 52 nM.
Aufgrund der Größe dieses Moleküls dürfte diese Verbindung nur schwer oral bioverfügbar sein. Es ist nicht unwahrscheinlich, dass das Molekül mit anderen NAD(P)(H)-abhängigen Enzymen eine Kreuzreaktion eingeht.

Die 17β-HSD 1 ist vor dem bekannten Stand der Technik ein Target für die lokale Hemmung der Estradiol-Biosynthese. Begleitend zur Behandlung mit Antihormonen, die die Bindung des aktiven Steroids am entsprechenden Rezeptor unterbinden sollen, können 17β-HSD 1 - Hemmer unterstützend zur Behandlung von Estrogen-abhängigen Erkrankungen eingesetzt werden.

Aufgabe der vorliegenden Erfindung ist es daher, weitere Verbindungen bereitzustellen, die eine selektive Hemmung der 17β-HSD 1 bewirken. Diese Verbindungen sollen zur Behandlung Estrogen-abhängiger Erkrankungen sowie hormonabhängiger Tumorerkrankungen geeignet sein, die sich durch Hemmung der 17β-Hydroxysteroiddehydrogenase Typ 1 beeinflussen lassen.

Weitere Gegenstände vorliegender Erfindung sind die Herstellung und Verwendung dieser Verbindungen als Arzneimittel zur Behandlung Estrogen-abhängiger Erkrankungen sowie hormonabhängiger Tumorerkrankungen, die sich durch Hemmung der 17β-Hydroxysteroiddehydrogenase Typ 1 beeinflussen lassen.

Verbindung 1 (siehe Beispiel 1) wird als Zwischenprodukt in WO-A-2004/074309 bekanntgemacht.

Die Aufgabe wird gemäß vorliegender Erfindung durch die Bereitstellung neuer 2-substituierter D-Homo-Estra-1,3,5(10)-triene der allgemeinen Formel I gelöst 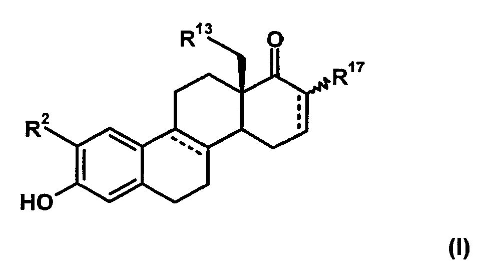 worin
- R²: eine gesättigte oder ungesättigte C₁-C₈-Alkylgruppe, einen Aralkyl- oder einen Alkylarylrest, eine C₁-C₈-Alkyloxygruppe oder ein Halogenatom,
- R¹³: ein Wasserstoffatom oder eine Methylgruppe,
- R¹⁷: ein Wasserstoff- oder ein Fluoratom,

bedeuten, wobei die gestrichelten Linien im B- und D-Ring des Steroidmoleküls zusätzliche Doppelbindungen sein können, sowie ihre pharmazeutisch annehmbaren Salze mit Ausnahme der Verbindung 1 (siehe Beispiel 1).

Weiterhin umfasst die vorliegende Erfindung die neuen Verbindungen als pharmazeutische Wirkstoffe, deren Herstellung, ihre therapeutische Anwendung und pharmazeutische Darreichungsformen, die die neuen Substanzen enthalten.

Die erfindungsgemäßen Verbindungen der allgemeinen Formel (I) oder deren pharmazeutisch annehmbare Salze können für die Herstellung eines Arzneimittels, insbesondere zur Behandlung estrogen-abhängiger Erkrankungen sowie hormonabhängiger Tumorerkrankungen, die sich durch Hemmung der 17β-Hydroxysteroiddehydrogenase Typ 1 beeinflussen lassen, verwendet werden.

Es wurde festgestellt, dass die erfindungsgemäßen niedermolekularen 2-substituierten D-Homoestra-1,3,5(10)-triene stärker als bisher bekannte 17β-HSD 1-Inhibitoren eine selektive Hemmung der 17β-HSD 1-Enzymaktivität *in vitro* bewirken.

Wenn nicht näher definiert, handelt es sich im Sinne der vorliegenden Erfindung bei einem Arylrest, der gegebenenfalls substituiert sein kann, um einen Phenyl-, 1- oder 2-Naphthylrest, wobei der Phenylrest bevorzugt ist. Wenn nicht ausdrücklich erwähnt, schließt Aryl immer auch einen Heteroarylrest mit ein. Beispiele für einen Heteroarylrest sind der 2-, 3- oder 4-Pyridinyl-, der 2- oder 3-Furyl, der 2- oder 3-Thienyl, der 2- oder 3-Pyrrolyl-, der 2-, 4- oder 5-Imidazolyl-, der Pyrazinyl-, der 2-, 4- oder 5-Pyrimidinyl-oder 3- oder 4-Pyridazinylrest.

Als Substituenten für einen Aryl- oder Heteroarylrest seien zum Beispiel ein Methyl-, Ethyl-, Trifluormethyl- Pentafluorethyl-, Trifluormethylthio-, Methoxy-, Ethoxy-, Nitro-, Cyano-, Halogen- (Fluor, Chlor, Brom, lod), Hydroxy-, Amino-, Mono(C₁₋₈-alkyl)- oder Di(C₁₋₈-alkyl)amino, wobei beide Alkylgruppen identisch oder verschieden sind, Di(aralkyl)amino, wobei beide Aralkylgruppen identisch oder verschieden sind, erwähnt.

Die C₁-C₈-Alkylgruppen für R² können gesättigt oder ungesättigt und teilweise oder vollständig substituiert sein. Als Vertreter der gesättigten Alkylreste sind Methyl-, Ethyl-, n-Propyl-, iso-Propyl-, n-, iso-, oder tert.-Butyl, n-, iso- oder neo-Pentylgruppe, Hexyl, Heptyl oder Octyl zu nennen. Methyl-, Ethyl- und Propyl sind bevorzugt.
Als Vertreter für ungesättigte Alkylreste stehen Allyl und Vinyl.

Für den C₁-C₅-Alkoxyrest R² kann eine Methoxy-, Ethoxy-, n-Propoxy-, iso-Propoxy-, n-, iso-, oder tert.-Butoxy, n-, iso- oder neo-Pentoxygruppe stehen.

Für ein Halogen kann im Fall von R² ein Chlor-, lod- oder Bromatom stehen.

Bevorzugt gemäß vorliegender Erfindung sind Verbindungen der allgemeinen Formel I, in denen R² C₁-C₅-Alkoxy, C₁-C₅-Alkyl oder C₂-C₃-Alkenyl bzw. Brom oder Chlor und R¹³ ein Wasserstoffatom sind.

Die nachstehend genannten Verbindungen sowie deren Verwendung sind erfindungsgemäß bevorzugt:
2) 2-Ethoxy-17a-oxo-17a-homoestra-1,3,5(10)-trien-3-ol
3) 2-Chloro-17a-oxo-17a-homoestra-1,3,5(10)-trien-3-ol **2**
4) 2-Bromo-17a-oxo-17a-homoestra-1,3,5(10)-trien-3-ol **3**
5) 2-lodo-17a-oxo-17a-homoestra-1,3,5(10)-trien-3-ol **4**
6) 2-Chloro-17α-Fluoro-17a-oxo-17a-homoestra-1,3,5(10)-trien-3-ol **5a**
7) 2-Chloro-17β-Fluoro-17a-oxo-17a-homoestra-1,3,5(10)-trien-3-ol **5b**
8) 2-Bromo-17α-Fluoro-17a-oxo-17a-homoestra-1,3,5(10)-trien-3-ol
9) 2-Bromo-17β-Fluoro-17a-oxo-17a-homoestra-1,3,5(10)-trien-3-ol
10) 2-(2-Phenylethyl)-17a-oxo-17a-homoestra-1,3,5(10)-trien-3-ol **6**
11) 2-Allyl-17a-oxo-17a-homoestra-1,3,5(10)-trien-3-ol **7**
12) 2-Allyl-17α-Fluoro-17a-oxo-17a-homoestra-1,3,5(10)-trien-3-ol
13) 2-Allyl-17β-Fluoro-17a-oxo-17a-homoestra-1,3,5(10)-trien-3-ol
14) 2-Chloro-17a-oxo-17a-homoestra-1,3,5(10),16-tetraen-3-ol
15) 2-Bromo-17a-oxo-17a-homoestra-1,3,5(10),16-tetraen-3-ol **8**
16) 2-Allyl-17a-oxo-17a-homoestra-1,3,5(10),16-tetraen-3-ol
17) 2-Propyl-17a-oxo-17a-homoestra-1,3,5(10)-trien-3-ol
18) 2-Methoxy-17a-oxo-17a-homo-18a-homoestra-1,3,5(10)-trien-3-ol
19) 2-Ethoxy-17a-oxo-17a-homo-18a-homoestra-1,3,5(10)-trien-3-ol
20) 2-Chloro-17a-oxo-17a-homo-18a-homoestra-1,3,5(10)-trien-3-ol
21) 2-Bromo-17a-oxo-17a-homo-18a-homoestra-1,3,5(10)-trien-3-ol
22) 2-lodo-17a-oxo-17a-homo-18a-homoestra-1,3,5(10)-trien-3-ol
23) 2-Chloro-17α-Fluoro-17a-oxo-17a-homo-18a-homoestra-1,3,5(10)-trien-3-ol
24) 2-Chloro-17β-Fluoro-17a-oxo-17a-homo-18a-homoestra-1,3,5(10)-trien-3-ol
25) 2-Bromo-17α-Fluoro-17a-oxo-17a-homo-18a-homoestra-1,3,5(10)-trien-3-ol
26) 2-Bromo-17β-Fluoro-17a-oxo-17a-homo-18a-homoestra-1,3,5(10)-trien-3-ol
27) 2-Allyl-17a-oxo-17a-homo-18a-homoestra-1,3,5(10)-trien-3-ol
28) 2-Allyl-17a-Fluoro-17a-oxo-17a-homo-18a-homoestra-1,3,5(10)-trien-3-ol
29) 2-Allyl-17β-Fluoro-17a-oxo-17a-homo-18a-homoestra-1,3,5(10)-trien-3-ol
30) 2-Chloro-17a-oxo-17a-homo-18a-homoestra-1,3,5(10),16-tetraen-3-ol
31) 2-Bromo-17a-oxo-17a-homo-18a-homoestra-1,3,5(10),16-tetraen-3-ol
32) 2-Allyl-17a-oxo-17a-homo-18a-homoestra-1,3,5(10),16-tetraen-3-ol
33) 2-Propyl-17a-oxo-17a-homo-18a-homoestra-1,3,5(10)-trien-3-ol

Verbindung 1 (siehe Beispiel 1) Zur Herstellung eines Arzneimittels und pharmazeutische Zusammensetzungen dieser verbindung sind auch von der vorliegenden Erfindung umfaßt.

### Pharmakologische Daten

### Hemmung der Aktivität der humanen 17β-Hydroxysteroiddehydrogenase Typ 1

Das Testverfahren ist in der Literatur gut beschrieben [Adamski J, Sierralta WD, Jungblut PW, Acta Endocrinol (Copenh.) 121(2) (1989), 161-7] und wird im Folgenden dargestellt.

Humane 17β-Hydroxysteroiddehydrogenasen (17β-HSDs) werden in *E. coli* Bakterien als His-Tag-Proteine oder als GST-Fusionsproteine überexprimiert. Die Suspensionen der Bakterienpellets in isotoner Kochsalzlösung werden für die Ermittlung der Enzymaktivitäten der 17β-HSDs bzw. zur Untersuchung deren Beeinflussung durch potentielle Inhibitoren (Estrogenderivate) eingesetzt.
Die Messungen erfolgen in Doppelbestimmung und bei Bedarf bei verschiedenen Konzentrationen an potentiellen Inhibitoren (z.B. bei Bestimmung der IC₅₀-Werte). Neben dem Zielenzym 17β-HSD1 werden andere Steroid-metabolisierende Enzyme in den Test einbezogen, um Kreuzreaktivitäten der Estrogenderivate zu untersuchen.

Zu einem definierten Volumen 100 mM Natriumphosphat-Puffer werden ³H-markiertes Substrat, Bakteriensuspension, DMSO (im Kontrollansatz; final 1%) oder die potentiellen Inhibitoren (Estronderivate in DMSO) sowie geeigneter Cofaktor (NADP(H) oder NAD(H); 5 mg/ml in H₂O) gegeben. Die Inkubation der Proben erfolgt bei 37°C im Wasserbad unter Schütteln, so dass in der Kontrolle (ohne zu testende Substanzen) ein Umsatz von etwa 30% erreicht wird.
Die Trennung von radioaktiv markiertem Substrat und Produkt erfolgt anschließend, nach Extraktion über 1 ml reversed phase-(RP-18)-Kartuschen, mittels HPLC auf einer RP18-Säule mit Acetonitril:Wasser 1:1 (v/v) als mobiler Phase. Die Radioaktivität wird mit Hilfe eines Durchfluss-Szintillationszählers detektiert.
Die Auswertung des Substratumsatzes mit und ohne zu testende Substanzen wird durch die Integration der Substrat- und Produkt-Peaks durchgeführt. Der Umsatz der Kontrolle wird auf 100% Umsatz normalisiert.

**Tab. 1 Hemmung der humanen 17β-Hydroxysteroiddehydrogenase**

| **Struktur** | **17**β**HSD1** |
|---|---|
| | **IC₅₀ [nM]** |
| 1 | 85 |
| 2 | 77 |
| 3 | 52 |
| 4 | 52 |
| 5a | 87 |
| 5b | 126 |
| 6 | 15 |
| 7 | 24 |
| Estron | 109 |

### Dosierung

Im allgemeinen sind zufriedenstellende Resultate bei der Behandlung estrogen-abhängiger Erkrankungen sowie hormonabhängiger Tumorerkrankungen zu erwarten, wenn die täglichen Dosen einen Bereich von 5 µg bis 50 mg der erfindungsgemäßen Verbindung pro kg Körpergewicht umfassen. Bei größeren Säugetieren, beispielsweise dem Menschen, liegt eine empfohlene tägliche Dosis im Bereich von 10 µg bis 30 mg pro kg Körpergewicht.

Geeignete Dosierungen für die erfindungsgemäßen Verbindungen betragen von 0,005 bis 50 mg pro Tag pro kg Körpergewicht, je nach Alter und Konstitution des Patienten, wobei die notwendige Tagesdosis durch Einmal- oder Mehrfachabgabe appliziert werden kann.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektion- und Infusionszubereitungen möglich.

Für die intraartikuläre Injektion können entsprechend zubereitete Kristallsuspensionen verwendet werden.

Für die intramuskuläre Injektion können wässrige und ölige Injektionslösungen oder Suspensionen und entsprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen als auch zur lokalen Therapie verwendet werden.

Zur pulmonalen Applikation der neuen Verbindungen können diese in Form von Aerosolen und Inhalaten verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel I sollte in diesen Zubereitungen 0,01% - 20% betragen, um eine ausreichende pharmakologische Wirkung zu erzielen.

Die vorliegende Erfindung umfasst die Verbindungen der allgemeinen Formel I sowie deren Verwendung zur Herstellung eines Arzneimittels, insbesondere zur Behandlung von estrogenabhängigen Erkrankungen, die sich durch die Hemmung der 17β-Hydroxysteroiddehydrogenase positiv beeinflussen lassen.

Die erfindungsgemäßen Verbindungen der allgemeinen Formel I werden bevorzugt zur Herstellung eines Arzneimittels zur Behandlung von hormonabhängigen Tumorerkrankungen der männlichen und weiblichen Keimdrüsen, männlichen und weiblichen Sexualorgane einschließlich der Brustdrüsen, insbesondere von Prostatakarzinomen oder Mammakarzinomen verwendet.

Weiterhin sind die erfindungsgemäßen Verbindungen zur Herstellung eines Arzneimittels zur Behandlung der Endometriose bevorzugt.

Ferner betrifft die vorliegende Erfindung pharmazeutische Zusammensetzungen, die mindestens eine erfindungsgemäße Verbindung, gegebenenfalls in Form eines pharmazeutisch/ pharmakologisch verträglichen Salzes, ohne oder zusammen mit pharmazeutisch verträglichen Hilfs- und/oder Trägerstoffen enthalten.

Diese pharmazeutischen Zusammensetzungen und Arzneimittel können zur oralen, rektalen, vaginalen, subkutanen, perkutanen, intravenösen oder intramuskulären Applikation vorgesehen sein. Sie enthalten neben üblichen Träger- und/ oder Verdünnungsmitteln mindestens eine erfindungsgemäße Verbindung.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Die pharmazeutischen Zusammensetzungen, die mindestens eine der erfindungsgemäßen Verbindungen enthalten, werden bevorzugt oral appliziert.

Es kommen auch parenterale Zubereitungen wie Injektionslösungen in Betracht. Weiterhin seien als Zubereitungen beispielsweise auch Suppositorien und Mittel zur vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylactat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen der erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltenden Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die erfindungsgemäßen Verbindungen können zur Prophylaxe und zur Therapie von Mamma- oder Prostatakarzinomen bzw. Endometriose mit einem oder mehreren der folgenden Wirkstoffe kombiniert verabreicht werden:
1) Antiandrogene wie Cyproteronacetat, Flutamid, Casodex etc.
2) Gonadrotropinhormon (GnRH) Agonisten wie Synarel, Lupron, Busrelin
3) Aromatasehemmer wie Anastrozol, Formestan, Letrozol, Exemestan
4) 5α-Reduktase Hemmer wie Finasterid
5) Zytostatika wie Vinblastin, Daunorubicin
6) VEGF-Kinase-Inhibitoren
7) Antigestagene wie Onapristone, Mifepristone
8) Antiestrogene wie Tamoxifen
9) Antisense Oligonukleotide
10) EGF-Antikörper

Darüber hinaus können die erfindungsgemäßen Verbindungen der allgemeinen Formel I zur Therapie und Prophylaxe weiterer oben nicht genannter Krankheitszustände eingesetzt werden.

Die nachfolgenden Beispiele dienen der näheren Erläuterung des Erfindungsgegenstandes, ohne ihn auf diese beschränken zu wollen.

Ausgehend von den 17-Keto-Derivaten können 17-Oxirane (M. Hübner, I. Noack, J. prakt. Chem. 1972, 314, 667), und daraus die entsprechenden 17a-Homo-Derivate (M. Hübner, K. Ponsold, Z. Chem. 1982, 22, 186) hergestellt werden. Die genannten Reaktionschritte können vor oder nach Funktionalisierung der 2-Position durchgeführt werden.

Die Einführung von Halogenen in die 2-Position erfolgt mittels *ortho*-Thallierung wie in der Literatur für 17-Keto-Derivate beschrieben (P.C. Bulman Page et al., Tetrahedron 1990, 46, 2059).
Die Funktionalisierung des C-Atoms 2 eines Estra-1,3,5(10)-trien-17-on-derivates erfolgt vorzugsweise durch Friedel-Crafts-Acylierung wie in der Literatur beschrieben (T. Nambara et al., Chem. Pharm. Bull. 1979, 18, 474). Nach Wechsel der Schutzgruppe in Position 3 wird durch Baeyer-Villiger-Oxidation ein 2-Carboxy-estra-1,3,5(10)-trien-17-on generiert (M.B. Smith, J. March, March's Advanced Organic Chemistry, 5. Edition, Wiley Sons 2001, 1417-1418). Der Ester wird verseift und mit dem entsprechenden Alkylhalogenid unter basischen Bedingungen in einen 2-Alkylether überführt. Die Spaltung der Schutzgruppe in Position 3 erfolgt wie in der Literatur beschrieben (T.W. Greene, P.G.M. Wuts, Protective Groups in Organic Synthesis, Wiley & Sons, 1999, 249-275). Dieses Verfahren oder andere (P.N. Rao, J.W. Cessac, Steroids 2002, 67, 1065) sind entsprechend auf die 17a-Homo-Derivate anwendbar.

Aus den 2-Acylderivaten können durch Reduktion mit Natriumborhydrid, Hydrierung und anschließender Oppenauer-Oxidation (C. Djerassi, Org. React. 1951, 6, 207) die entsprechenden 2-Alkylderivate hergestellt werden.

Die Einführung der 2-Allylgruppe kann über eine Claisen-Umlagerung wie in der Literatur beschrieben (L. Troisi et al., Tetrahedron Lett. 1999, 40, 1771) erfolgen. Nachfolgend kann analog der von T. Buskas et al. (J. Org. Chem. 2000, 65, 958) beschriebenen Methoden weiter funktionalisiert werden.

Längere, auch ungesättigte oder funktionalisierte Alkylreste in 2-Position können über die Sonogashira-Kupplung geeigneter, evtl. entsprechend geschützter Alkine und nachfolgender (partieller) Hydrierung erhalten werden.

Die 17-Fluorierung des 17a-Ketons kann wie von A.C. Stalford et al. (Steroids 1997, 62, 750) oder auch S. Stavber et al. (Synthesis 2002, 2609) beschrieben durchgeführt werden.

Die Synthese von 16,17-Dehydroderivaten kann ebenfalls analog bekannter Verfahren (siehe z.B. P.N. Rao et al., Steroids 2002, 67, 1079) durchgeführt werden.

### Herstellungsverfahren

### Beispiel 1

### 2-Methoxy-17a-oxo-17a-homoestra-1,3,5(10)-trien-3-ol 1

3.61 g 17α-Azidomethyl-3,17β-dihydroxy-2-methoxy-estra-1,3,5(10)-trien und 7.5 g Natriumiodid wurden in 250 mL Acetonitril suspendiert und bei Raumtemperatur langsam mit 15 mL Trimethylsilylchlorid versetzt. Nach 4h wurden weitere 4 mL Trimethylsilylchlorid zugegeben und nach weiteren 2.5h wurde mit ges. Natriumthiosulfatlsg. und Wasser versetzt und mit Dichlormethan extrahiert (3x). Die vereinigten organischen Phasen wurden mit wässr. Natriumbicarbonatlsg. gewaschen, getrocknet und am Rotationsverdampfer eingeengt. Flashchromatographie (Cyclohexan/Essigester = 10:1 → 7:1 → 5:1) lieferte 2.12 g (67%) 2-Methoxy-17a-oxo-17a-homoestra-1,3,5(10)-trien-3-ol **1** als farblose Kristalle.
¹H-NMR (CDCl₃): δ = 1.13 (s, 3H; 18-CH₃), 2.62-2.71 (m, 1H; 17-H), 2.77 (dd, 2H; 6-CH₂), 3.86 (s, 3H; 2-OCH₃), 5.48 (s, 1H; 3-OH), 6.63, 6.78 (2 s, 2H; 1-H, 4-H).

### Beispiel 2

### 2-Chloro-17a-oxo-17a-homoestra-1,3,5(10)-trien-3-ol 2

307 mg (851 µmol) 3-Acetoxy-2-chloro-17a-oxo-17a-homoestra-1,3,5(10)-trien wurden in 24 mL Dichlormethan/Methanol (2:1) gelöst und mit 45 mg Natriummethanolat versetzt. Nach 1.5h wurde mit Amberlite IR120 (H+) neutralisiert, gefiltert und am Rotationsverdampfer eingeengt. Flashchromatographie (Toluol/Essigester = 25:1) lieferte 173 mg (64%) 2-Chloro-17a-oxo-17a-homoestra-1,3,5(10)-trien-3-ol **2** als farblose Kristalle.
¹H-NMR (CDCl₃): δ = 1.12 (s, 3H; 18-CH₃), 2.62-2.82 (m, 2H; 17-H, 6-CH₂), 5.39 (s, 1H; 3-OH), 6.72, 7.21 (2 s, 2H; 1-H, 4-H).

### Beispiel 3

### 2-Bromo-17a-oxo-17a-homoestra-1,3,5(10)-trien-3-ol 3

**3** wurde analog der für die 17-Ketoderivate beschriebenen Methode von P.C. Bulman Page et al., Tetrahedron 1990, 46, 2059 hergestellt.
¹H-NMR (CDCl₃): δ = 1.12 (s, 3H; 18-CH₃), 2.62-2.82 (m, 2H; 17-H, 6-CH₂), 5.41 (s, 1H; 3-OH), 6.73, 7.34 (2 s, 2H; 1-H, 4-H).

### Beispiel 4

### 2-lodo-17a-oxo-17a-homoestra-1,3,5(10)-trien-3-ol 4

**4** wurde analog der für die 17-Ketoderivate beschriebenen Methode von P.C. Bulman Page et al., Tetrahedron 1990, 46, 2059 hergestellt.
¹H-NMR (CDCl₃): δ = 1.12 (s, 3H; 18-CH₃), 2.62-2.71 (ddd, J = 6.8, 14.1, 14.1 Hz, 1H; 17-H), 2.77-2.81 (m, 2 H; 6-CH₂), 5.29 (s, 1H; 3-OH), 6.71, 7.52 (2 s, 2H; 1-H, 4-H).

### Beispiel 5

### 2-Chloro-17α-Fluoro-17a-oxo-17a-homoestra-1,3,5(10)-trien-3-ol 5a und 2-Chloro-17β-Fluoro-17a-oxo-17a-homoestra-1,3,5(10)-trien-3-ol 5b

60 mg (188 µmol) 2-Chloro-17a-oxo-17a-homoestra-1,3,5(10)-trien-3-ol 2 wurden mit etwa 190 mg 1-Fluor-4-hydroxy-1,4-diazonia-bicyclo[2,2,2]-octan bis(tetrafluoroborat) auf Aluminiumoxid in 10 mL Methanol für 5h am Rückfluß erhitzt, auf Raumtemperatur abgekühlt, mit 1 N Salzsäure versetzt und mit Dichlormethan extrahiert. Die organischen Phasen werden getrocknet und am Rotationsverdampfer eingeengt. Reinigung mittels HPLC (Chiracel OJ-H, n-Heptan/2-Propanol = 6:4) lieferte je etwa 10 mg 2-Chloro-17α-Fluoro-17a-oxo-17a-homoestra-1,3,5(10)-trien-3-ol **5a** und 2-Chloro-17β-Fluoro-17a-oxo-17a-homoestra-1,3,5(10)-trien-3-ol **5b** als farblose Feststoffe.
**5a:** ¹H-NMR (CDCl₃): δ = 1.13 (s, 3H; 18-CH₃), 5.32 (ddd, *J_{HF}* = 48.8, *J_{HH}* = 7.4, 12.5 Hz, 1H; 17β-H), 6.72, 7.21 (2 s, 2H; 1-H, 4-H) - ¹⁹F-NMR (CDCl₃): δ = - 192.20 (dd, *J* = 48.9, 12.8 Hz).
**5b:** ¹H-NMR (CDCl₃): δ = 1.21 (d, *J* = 3.1 Hz, 3H; 18-CH₃), 4.86 (ddd, *J_{HF}* = 49.2, *J_{HH}* = 3.9, 6.6 Hz, 1H; 17α-H), 6.73, 7.20 (2 s, 2H; 1-H, 4-H) - ¹⁹F-NMR (CDCl₃): δ = - 183.02 - -183.28 (m).

### Beispiel 6

### 2-(2-Phenylethyl)-17a-oxo-17a-homoestra-1,3,5(10)-trien-3-ol 6

56 mg (123 µmol) 3-Acetoxy-2-iodo-17a-oxo-17a-homoestra-1,3,5(10)-trien wurden in 8 mL Triethylamin/THF (3:1) gelöst, mit 3 mg Palladium-II-acetat, 5 mg Kupfer-l-iodid, 6.5 mg Triphenylphosphin und 26 µL Phenylacetylen versetzt und 45 min bei Raumtemperatur unter Argon gerührt. Anschließend wurde mit Essigester verdünnt, mit 1 N Salzsäure, ges. Natriumhydrogencarbonatlösung und Kochsalzlösung gewaschen, getrocknet, am Rotationsverdampfer eingeengt und flashchromatographisch gereinigt (Cyclohexan/Essigester 10:1 → 5:1). Man erhielt 42 mg (80%) 3-Acetoxy-2-(2-phenylethinyl)-17a-oxo-17a-homoestra-1,3,5(10)-trien als braunschwarze Kristalle.
¹H-NMR (CDCl₃): δ = 1.12 (s, 3H; 18-CH₃), 2.35 (s, 3H, COCH₃), 6.82, 7.50 (2 s, 2H; 1-H, 4-H), 7.31-7.48 (m, 5H; Ph) - ¹³C-NMR (CDCl₃): δ = 16.79, 20.84, 22.87, 25.59, 25.80, 26.22, 29.75, 32.33, 37.08, 38.14, 42.89, 48.17, 50.17, 84.58, 92.92, 114.101, 121,64, 122.91, 128.12, 129.82, 131.24, 137.92, 138.52, 148.90, 168.97, 215.83. 27 mg (63µmol) 3-Acetoxy-2-(2-phenylethinyl)-17a-oxo-17a-homoestra-1,3,5(10)-trien wurde in 10 mL Essigester/THF (9:1) gelöst, mit 3 Tropfen Essigsäure und 55 mg Palladium auf Aktivkohle (10%ig) versetzt und 3h hydriert. Anschließend wurde der Katalysator abfiltriert, am Rotationsverdampfer eingeengt und mehrmals mit Toluol coevaporiert. Der Rückstand wurde in 6 mL Dichlormethan gelöst, mit 20 mL Methanol und 100 mg Natriummethanolat versetzt und 2h gerührt. Danach wurde mit Amberlite IR120 (H+) neutralisiert, gefiltert und am Rotationsverdampfer eingeengt. Flashchromatographie (Cyclohexan/Essigester = 5:1) lieferte 16 mg (65%) 2-(2-Phenylethyl)-17a-oxo-17a-homoestra-1,3,5(10)-trien-3-ol 6 als farblose Nadeln.
¹H-NMR (CDCl₃): δ = 1.13 (s, 3H; 18-CH₃), 2.85-2.89 (m, 4H, PhCH₂), 4.46 (s, 1H; OH), 6.48, 7.02 (2 s, 2H; 1-H, 4-H), 7.19-7.30 (m, 5H; Ph).

### Beispiel 7

### 2-Allyl-17a-oxo-17a-homoestra-1,3,5(10)-trien-3-ol 7

315 mg (1.11 mmol) 17a-Oxo-17a-homoestra-1,3,5(10)-trien-3-ol wurden in 5 mL abolutem DMF gelöst, mit 1.8 g Cäsiumcarbonat und 1 mL Allylbromid versetzt und für 3h auf 60°C erhitzt. Anschließend wird mit Essigester versetzt und mit Wasser und Kochsalzlösung gewaschen. Die organische Phase wurde abgetrennt, getrocknet und am Rotationsverdampfer eingeengt. Flashchromatographie (Cyclohexan/Essigester = 40:1 → 20:1 → 10:1) lieferte 322 mg (89%) 3-Allyloxy-17a-oxo-17a-homoestra-1,3,5(10)-trien als farblose Kristalle.
¹H-NMR (CDCl₃): δ = 1.12 (d, 3H; 18-CH₃), 2.62-2.71 (m, 1H; 17-H), 2.83-2.86 (m, 2H; 6-H), 4.50 (d, *J* = 5.0 Hz, 2H; OCH₂), 5.26 (dd, *J* = 1.2, 10.5 Hz, 1H, =CHH), 5.37 (dd, *J* = 1.2, 17.2 Hz, 1H; =CH*H*), 5.99-6.07 (m, 1H; C*H*=CH₂), 6.63 (d, *J* = 2.3 Hz, 1H; 4-H), 6.72 (dd, *J* = 2.3, 8.2 Hz, 1H; 2-H), 7.20 (d, *J* = 8.6 Hz, 1H; 1-H).

315 mg (971 µmol) 3-Allyloxy-17a-oxo-17a-homoestra-1,3,5(10)-trien wurden in 25 mL Diethylanilin unter Argon gelöst und für 8h am Rückfluß erhitzt. Anschließend wurde mit Essigester versetzt und mit 1 N Salzsäure und Kochsalzlösung gewaschen. Die organische Phase wurde abgetrennt, getrocknet und am Rotationsverdampfer eingeengt. Flashchromatographie (Cyclohexan/Essigester = 9:1) lieferte 312 mg (99%) einer Mischung der beiden Regioisomeren als farblosen Schaum. Aufrennung mittels HPLC (Chirapak AD-H, n-Heptan/2-Propanol = 8:2) lieferte 108 mg 2-Allyl-17a-oxo-17a-homoestra-1,3,5(10)-trien-3-ol **7** als farblose Kristalle.
¹H-NMR (CDCl₃): δ = 1.12 (d, 3H; 18-CH₃), 2.62-2.71 (m, 1H; 17-H), 2.78-2.82 (m, 2H; 6-H), 3.37 (d, *J* = 6.3 Hz, 2H; OC*H*₂CH=), 4.89 (s, 1H; OH), 5.12-5.18 (m, 2H, =C*H*₂), 5.94-6.05 (m, 1H; C*H*=CH₂), 6.54, 7.02 (2 s, 2H; 1-H, 4-H).

### Beispiel 8

### 2-Bromo-17a-oxo-17a-homoestra-1,3,5(10),16-tetraen-3-ol 8

¹H-NMR (CDCl₃): δ = 1.05 (d, 3H; 18-CH₃), 2.62-2.71 (m, 1H; 17-H), 5.95 (dd, *J* = 2.5, 10.0 Hz, 1H; =CH), 6.87-6.91 (m, 1H, =CH), 6.74, 7.36 (2 s, 2H; 1-H, 4-H) - ¹³C-NMR (CDCl₃): δ = 15.63, 25.64, 25.85, 27.13, 29.28, 32.08, 38.81, 42.34, 44.45, 45.32, 107.42, 115.37, 127.56, 128.61, 133.76, 137.46, 147.25, 149.79, 205.04.

## Patentansprüche

1. 2-substituierte D-Homo-Estratriene der allgemeinen Formel I, 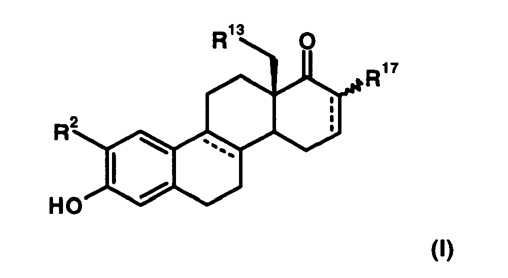 worin
R² eine gesättigte oder ungesättigte C₁-C₈-Alkylgruppe, einen Aralkyl- oder einen Alkylarylrest, eine C₁-C₈-Alkyloxygruppe oder ein Halogenatom,
R¹³ ein Wasserstoffatom oder eine Methylgruppe,
R¹⁷ ein Wasserstoff- oder ein Fluoratom,
bedeuten, wobei die gestrichelten Linien im B- und D-Ring des Steroidmoleküls zusätzliche Doppelbindungen sein können sowie ihre pharmazeutisch annehmbaren Salze mit Ausnahme der Verbindung 2-methoxy-17a-oxo-17a-homoestra-1,3,5(10)-trien-3-ol.

2. 2-substituierte D-Homo-Estra-1,3,5(10)-triene nach Anspruch 1, **dadurch gekennzeichnet, dass** R² ein C₁-C₅-Alkoxy, C₁-C₅-Alkyl oder C₂-C₃-Alkenyl bzw. Brom oder Chlor ist.

3. 2-substituierte D-Homo-Estra-1,3,5(10)-triene nach Anspruch 1, **dadurch gekennzeichnet, dass** R² ein Methoxy- oder Ethoxy-, ein Methyl-, Ethyl- oder Propylsowie ein Allylrest ist.

4. 2-substituierte D-Homo-Estra-1,3,5(10)-triene nach Anspruch 1, **dadurch gekennzeichnet, dass** R¹³ ein Wasserstoffatom ist.

5. 2-substituierte D-Homoestra-1,3,5(10)-triene nach Anspruch 1, nämlich
2) 2-Ethoxy-17a-oxo-17a-homoestra-1,3,5(10)-trien-3-ol
3) 2-Chloro-17a-oxo-17a-homoestra-1,3,5(10)-trien-3-ol **2**
4) 2-Bromo-17a-oxo-17a-homoestra-1,3,5(10)-trien-3-ol **3**
5) 2-lodo-17a-oxo-17a-homoestra-1,3,5(10)-trien-3-ol **4**
6) 2-Chloro-17α-Fluoro-17a-oxo-17a-homoestra-1,3,5(10)-trien-3-ol **5a**
7) 2-Chloro-17β-Fluoro-17a-oxo-17a-homoestra-1,3,5(10)-trien-3-ol **5b**
8) 2-Bromo-17α-Fluoro-17a-oxo-17a-homoestra-1,3,5(10)-trien-3-ol
9) 2-Bromo-17β-Fluoro-17a-oxo-17a-homoestra-1,3,5(10)-trien-3-ol
10) 2-(2-Phenylethyl)-17a-oxo-17a-homoestra-1,3,5(10)-trien-3-ol **6**
11) 2-Allyl-17a-oxo-17a-homoestra-1,3,5(10)-trien-3-ol **7**
12) 2-Allyl-17α-Fluoro-17a-oxo-17a-homoestra-1,3,5(10)-trien-3-ol
13) 2-Allyl-17β-Fluoro-17a-oxo-17a-homoestra-1,3,5(10)-trien-3-ol
14) 2-Chloro-17a-oxo-17a-homoestra-1,3,5(10),16-tetraen-3-ol
15) 2-Bromo-17a-oxo-17a-homoestra-1,3,5(10),16-tetraen-3-ol **8**
16) 2-Allyl-17a-oxo-17a-homoestra-1,3,5(10),16-tetraen-3-ol
17) 2-Propyl-17a-oxo-17a-homoestra-1,3,5(10)-trien-3-ol
18) 2-Methoxy-17a-oxo-17a-homo-18a-homoestra-1,3,5(10)-trien-3-ol
19) 2-Ethoxy-17a-oxo-17a-homo-18a-homoestra-1,3,5(10)-trien-3-ol
20) 2-Chloro-17a-oxo-17a-homo-18a-homoestra-1,3,5(10)-trien-3-ol
21) 2-Bromo-17a-oxo-17a-homo-18a-homoestra-1,3,5(10)-trien-3-ol
22) 2-Iodo-17a-oxo-17a-homo-18a-homoestra-1,3,5(10)-trien-3-ol
23) 2-Chloro-17α-Fluoro-17a-oxo-17a-homo-18a-homoestra-1,3,5(10)-trien-3-ol
24) 2-Chloro-17β-Fluoro-17a-oxo-17a-homo-18a-homoestra-1,3,5(10)-trien-3-ol
25) 2-Bromo-17α-Fluoro-17a-oxo-17a-homo-18a-homoestra-1,3,5(10)-trien-3-ol
26) 2-Bromo-17β-Fluoro-17a-oxo-17a-homo-18a-homoestra-1,3,5(10)-trien-3-ol
27) 2-Allyl-17a-oxo-17a-homo-18a-homoestra-1,3,5(10)-trien-3-ol
28) 2-Allyl-17α-Fluoro-17a-oxo-17a-homo-18a-homoestra-1,3,5(10)-trien-3-ol
29) 2-Allyl-17β-Fluoro-17a-oxo-17a-homo-18a-homoestra-1,3,5(10)-trien-3-ol
30) 2-Chloro-17a-oxo-17a-homo-18a-homoestra-1,3,5(10),16-tetraen-3-ol
31) 2-Bromo-17a-oxo-17a-homo-18a-homoestra-1,3,5(10),16-tetraen-3-ol
32) 2-Allyl-17a-oxo-17a-homo-18a-homoestra-1,3,5(10),16-tetraen-3-ol
33)2-Propyl-17a-oxo-17a-homo-18a-homoestra-1,3,5(10)-trien-3-ol

6. 2-substituierte D-Homoestra-1,3,5(10)-triene nach einem der Ansprüche 1 bis 5 oder 2-methoxy-17a-oxo-17a-homoestra-1,3,5(10)-trien-3-ol zur Herstellung eines Arzneimittels.

7. Verwendung von 2-substituierten D-Homoestra-1,3,5(10)-trienen nach einem der Ansprüche 1-5 oder 2-methoxy-17a-oxo-17a-homoestra-1,3,5(10)-trien-3-ol zur Herstellung eines Arzneimittels zur Prophylaxe und Therapie von estrogenabhängigen Erkrankungen, die sich durch die Hemmung der 17β-Hydroxysteroiddehydrogenase positiv beeinflussen lassen.

8. Verwendung von 2-substituierten D-Homoestra-1,3,5(10)-trienen nach Anspruch 7, wobei zumindest ein weiterer Wirkstoff in Kombination zur Herstellung eines Arzneimittels verwendet wird.

9. Verwendung von 2-substituierten D-Homoestra-1,3,5(10)-trienen nach Anspruch 8, wobei der weitere Wirkstoff ein Antiandrogen, Antigestagen, Aromatasehemmer oder ein Antiestrogen ist.

10. Verwendung von 2-substituierten D-Homoestra-1,3,5(10)-trienen nach einem der Ansprüche 7 bis 9 zur Herstellung eines Arzneimittels zur Prophylaxe und Therapie von hormonabhängigen Tumorerkrankungen der männlichen und weiblichen Keimdrüsen, männlichen und weiblichen Sexualorgane einschließlich der Brustdrüsen.

11. Verwendung von 2-substituierten D-Homoestra-1,3,5(10)-trienen nach Anspruch 10 zur Herstellung eines Arzneimittels zur Prophylaxe und Therapie des Mammakarzinoms.

12. Verwendung von 2-substituierten D-Homoestra-1,3,5(10)-trienen nach Anspruch 10 zur Herstellung eines Arzneimittels zur Prophylaxe und Therapie des Prostatakarzinoms.

13. Verwendung von 2-substituierten D-Homoestra-1,3,5(10)-trienen nach Anspruch 10 zur Herstellung eines Arzneimittels zur Behandlung der Endometriose.

14. Pharmazeutische Zusammensetzungen enthaltend mindestens eine Verbindung der allgemeinen Formel I nach einem der Ansprüche 1 bis 5 oder 2-methoxy-17a-oxo-17a-homoestra-1,3,5(10)-trien-3-ol und ggf. zumindest einem weiteren Wirkstoff zusammen mit pharmazeutisch verträglichen Hilfs- und/oder Trägerstoffen, wobei der weitere Wirkstoff ein Antiandrogen, Antigestagen, Aromatasehemmer oder ein Antiestrogen ist.

## Claims

1. 2-Substituted D-homo-oestratrienes of the general formula I, 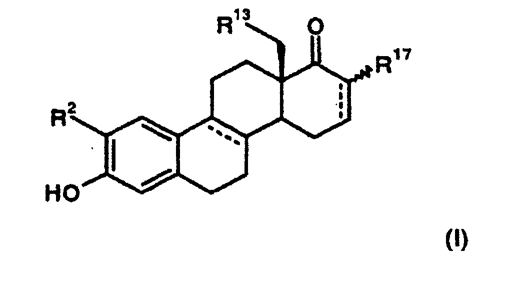 in which
R² is a saturated or unsaturated C₁-C₈-alkyl group, an aralkyl or an alkylaryl radical, a C₁-C₈-alkyloxy group or a halogen atom,
R¹³ is a hydrogen atom or a methyl group,
R¹⁷ is a hydrogen or a fluorine atom,
where the dashed lines in the B ring and D ring of the steroid molecule can be additional double bonds, and their pharmaceutically acceptable salts with the exception of the compound 2-methoxy-17a-oxo-17a-homo-oestra-1,3,5(10)-trien-3-ol.

2. 2-Substituted D-homo-oestra-1,3,5(10)-trienes according to Claim 1, **characterized in that** R² is a C₁-C₅-alkoxy, C₁-C₅-alkyl or C₂-C₃-alkenyl radical or is bromine or chlorine.

3. 2-Substituted D-homo-oestra-1,3,5(10)-trienes according to Claim 1, **characterized in that** R² is a methoxy or ethoxy radical, a methyl, ethyl or propyl radical, and an allyl radical.

4. 2-Substituted D-homo-oestra-1,3,5(10)-trienes according to Claim 1, **characterized in that** R¹³ is a hydrogen atom.

5. 2-Substituted D-homo-oestra-1,3,5(10)-trienes according to Claim 1, namely
2) 2-ethoxy-17a-oxo-17a-homo-oestra-1,3,5(10)-trien-3-ol
3) 2-chloro-17a-oxo-17a-homo-oestra-1,3,5(10)-trien-3-ol **2**
4) 2-bromo-17a-oxo-17a-homo-oestra-1,3,5(10)-trien-3-ol **3**
5) 2-iodo-17a-oxo-17a-homo-oestra-1,3,5(10)-trien-3-ol **4**
6) 2-chloro-17α-fluoro-17a-oxo-17a-homo-oestra-1,3,5(10)-trien-3-ol **5a**
7) 2-chloro-17β-fluoro-17a-oxo-17a-homo-oestra-1,3,5(10)-trien-3-ol **5b**
8) 2-bromo-17α-fluoro-17a-oxo-17a-homo-oestra-1,3,5(10)-trien-3-ol
9) 2-bromo-17β-fluoro-17a-oxo-17a-homo-oestra-1,3,5(10)-trien-3-ol
10) 2-(2-phenylethyl)-17a-oxo-17a-homo-oestra-1,3,5(10)-trien-3-ol **6**
11) 2-allyl-17a-oxo-17a-homo-oestra-1,3,5(10)-trien-3-ol **7**
12) 2-allyl-17α-fluoro-17a-oxo-17a-homo-oestra-1,3,5(10)-trien-3-ol
13) 2-allyl-17β-fluoro-17a-oxo-17a-homo-oestra-1,3,5(10)-trien-3-ol
14) 2-chloro-17a-oxo-17a-homo-oestra-1,3,5(10),16-tetraen-3-ol
15) 2-bromo-17a-oxo-17a-homo-oestra-1,3,5(10),16-tetraen-3-ol **8**
16) 2-allyl-17a-oxo-17a-homo-oestra-1,3,5(10),16-tetraen-3-ol
17) 2-propyl-17a-oxo-17a-homo-oestra-1,3,5(10)-trien-3-ol
18) 2-methoxy-17a-oxo-17a-homo-18a-homo-oestra-1,3,5(10)-trien-3-ol
19) 2-ethoxy-17a-oxo-17a-homo-18a-homo-oestra-1,3,5(10)-trien-3-ol
20) 2-chloro-17a-oxo-17a-homo-18a-homo-oestra-1,3,5(10)-trien-3-ol
21) 2-bromo-17a-oxo-17a-homo-18a-homo-oestra-1,3,5(10)-trien-3-ol
22) 2-iodo-17a-oxo-17a-homo-18a-homo-oestra-1,3,5(10)-trien-3-ol
23) 2-chloro-17α-fluoro-17a-oxo-17a-homo-18a-homo-oestra-1,3,5(10)-trien-3-ol
24) 2-chloro-17β-fluoro-17a-oxo-17a-homo-18a-homo-oestra-1,3,5(10)-trien-3-ol
25) 2-bromo-17α-fluoro-17a-oxo-17a-homo-18a-homo-oestra-1,3,5(10)-trien-3-ol
26) 2-bromo-17β-fluoro-17a-oxo-17a-homo-18a-homo-oestra-1,3,5(10)-trien-3-ol
27) 2-allyl-17a-oxo-17a-homo-18a-homo-oestra-1,3,5(10)-trien-3-ol
28) 2-allyl-17α-fluoro-17a-oxo-17a-homo-18a-homo-oestra-1,3,5(10)-trien-3-ol
29) 2-allyl-17β-fluoro-17a-oxo-17a-homo-18a-homo-oestra-1,3,5(10)-trien-3-ol
30) 2-chloro-17a-oxo-17a-homo-18a-homo-oestra-1,3,5(10),16-tetraen-3-ol
31) 2-bromo-17a-oxo-17a-homo-18a-homo-oestra-1,3,5(10),16-tetraen-3-ol
32) 2-allyl-17a-oxo-17a-homo-18a-homo-oestra-1,3,5(10),16-tetraen-3-ol
33) 2-propyl-17a-oxo-17a-homo-18a-homo-oestra-1,3,5(10)-trien-3-ol

6. 2-Substituted D-homo-oestra-1,3,5(10)-trienes according to one of Claims 1 to 5 or 2-methoxy-17a-oxo-17a-homo-oestra-1,3,5(10)-trien-3-ol for the production of a medicament.

7. Use of 2-substituted D-homo-oestra-1,3,5(10)-trienes according to one of Claims 1 to 5 or 2-methoxy-17a-oxo-17a-homo-oestra-1,3,5(10)-trien-3-ol for the production of a medicament for the prophylaxis and therapy of oestrogen-dependent diseases that can be positively influenced by the inhibition of 17β-hydroxysteroid dehydrogenase.

8. Use of 2-substituted D-homo-oestra-1,3,5(10)-trienes according to Claim 7, at least one further active compound being used in combination for the production of a medicament.

9. Use of 2-substituted D-homo-oestra-1,3,5(10)-trienes according to Claim 8, the further active compound being an antiandrogen, anti-gestagen, aromatase inhibitor or an anti-oestrogen.

10. Use of 2-substituted D-homo-oestra-1,3,5(10)-trienes according to one of Claims 7 to 9 for the production of a medicament for the prophylaxis and therapy of hormone-dependent tumours of the male and female gonads, and male and female sex organs including the mammary glands.

11. Use of 2-substituted D-homo-oestra-1,3,5(10)-trienes according to Claim 10 for the production of a medicament for the prophylaxis and therapy of breast carcinoma.

12. Use of 2-substituted D-homo-oestra-1,3,5(10)-trienes according to Claim 10 for the production of a medicament for the treatment of prostate carcinoma.

13. Use of 2-substituted D-homo-oestra-1,3,5(10)-trienes according to Claim 10 for the production of a medicament for the treatment of endometriosis.

14. Pharmaceutical compositions comprising at least one compound of the general formula I according to one of Claims 1 to 5 or 2-methoxy-17a-oxo-17a-homooestra-1,3,5(10)-trien-3-ol and optionally at least one further active compound together with pharmaceutically tolerable excipients and/or vehicles, the further active compound being an antiandrogen, anti-gestagen, aromatase inhibitor or an anti-oestrogen.

## Revendications

1. D-homo-estratriènes substitués en 2ème position de formule générale I, 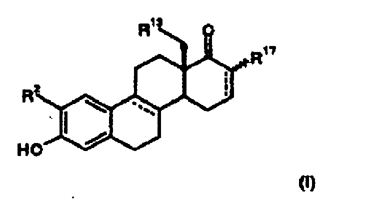 où
R² représente un groupe C₁-C₈-alkyle saturé ou insaturé, un radical aralkyle ou alkylaryle, un groupe C₁-C₈-alkyloxy ou un atome d'halogène,
R¹³ représente un atome d'hydrogène ou un groupe méthyle,
R¹⁷ représente un atome d'hydrogène ou de fluor,
où les lignes en pointillés dans le cycle B et D de la molécule de stéroïde peuvent être des doubles liaisons supplémentaires ainsi que leurs sels pharmaceutiquement acceptables à l'exception du composé 2-méthoxy-17a-oxo-17a-homoestra-1,3,5(10)-trién-3-ol.

2. D-homo-estra-1,3,5(10)-triènes substitués en 2ème position selon la revendication 1, **caractérisés en ce que** R² représente un radical C₁-C₅-alcoxy, C₁-C₅-alkyle ou C₂-C₃-alcényle ou brome ou chlore.

3. D-homo-estra-1,3,5(10)-triènes substitués en 2ème position selon la revendication 1, **caractérisés en ce que** R² représente un radical méthoxy ou éthoxy, méthyle, éthyle ou propyle ainsi qu'un radical allyle.

4. D-homo-estra-1,3,5(10)-triènes substitués en 2ème position selon la revendication 1, **caractérisés en ce que** R¹³ représente un atome d'hydrogène.

5. D-homoestra-1,3,5(10)-triènes substitués en 2ème position selon la revendication 1, notamment,
2) 2-éthoxy-17a-oxo-17a-homoestra-1,3,5(10)-trién-3-ol
3) 2-chloro-17a-oxo-17a-homoestra-1,3,5(10)-trién-3-ol 2
4) 2-bromo-17a-oxo-17a-homoestra-1,3,5(10)-trién-3-ol 3
5) 2-iodo-17a-oxo-17a-homoestra-1,3,5(10)-trién-3-ol 4
6) 2-chloro-17α-fluoro-17a-oxo-17a-homoestra-1,3,5(10)-trién-3-ol 5a
7) 2-chloro-17β-fluoro-17a-oxo-17a-homoestra-1,3,5(10)-trién-3-ol 5b
8) 2-bromo-17α-fluoro-17a-oxo-17a-homoestra-1,3,5(10)-trién-3-ol
9) 2-bromo-17β-fluoro-17a-oxo-17a-homoestra-1,3,5(10)-trién-3-ol
10) 2-(2-phényléthyl)-17a-oxo-17a-homoestra-1,3,5(10)-trién-3-ol 6
11) 2-allyl-17a-oxo-17a-homoestra-1,3,5(10)-trién-3-ol 7
12) 2-allyl-17α-fluoro-17a-oxo-17a-homoestra-1,3,5(10)-trién-3-ol
13) 2-allyl-17β-fluoro-17a-oxo-17a-homoestra-1,3,5(10)-trién-3-ol
14) 2-chloro-17a-oxo-17a-homoestra-1,3,5(10),16-tétraén-3-ol
15) 2-bromo-17a-oxo-17a-homoestra-1,3,5(10),16-tétraén-3-ol 8
16) 2-allyl-17a-oxo-17a-homoestra-1,3,5(10),16-tétraén-3-ol
17) 2-propyl-17a-oxo-17a-homoestra-1,3,5(10)-trién-3-ol
18) 2-méthoxy-17a-oxo-17a-homo-18a-homoestra-1,3,5(10)-trién-3-ol
19) 2-éthoxy-17a-oxo-17a-homo-18a-homoestra-1,3,5(10)-trién-3-ol
20) 2-chloro-17a-oxo-17a-homo-18a-homoestra-1,3,5(10)-trién-3-ol
21) 2-bromo-17a-oxo-17a-homo-18a-homoestra-1,3,5(10)-trién-3-ol
22) 2-iodo-17a-oxo-17a-homo-18a-homoestra-1,3,5(10)-trién-3-ol
23) 2-chloro-17α-fluoro-17a-oxo-17a-homo-18a-homoestra-1,3,5(10)-trién-3-ol
24) 2-chloro-17β-fluoro-17a-oxo-17a-homo-18a-homoestra-1,3,5(10)-trién-3-ol
25) 2-bromo-17α-fluoro-17a-oxo-17a-homo-18a-homoestra-1,3,5(10)-trién-3-ol
26) 2-bromo-17β-fluoro-17a-oxo-17a-homo-18a-homoestra-1,3,5(10)-trién-3-ol
27) 2-allyl-17a-oxo-17a-homo-18a-homoestra-1,3,5(10)-trién-3-ol
28) 2-allyl-17α-fluoro-17a-oxo-17a-homo-18a-homoestra-1,3,5(10)-trién-3-ol
29) 2-allyl-17β-fluoro-17a-oxo-17a-homo-18a-homoestra-1,3,5(10)-trién-3-ol
30) 2-chloro-17a-oxo-17a-homo-18a-homoestra-1,3,5(10),16-tétraén-3-ol
31) 2-bromo-17a-oxo-17a-homo-18a-homoestra-1,3,5(10),16-tétraén-3-ol
32) 2-allyl-17a-oxo-17a-homo-18a-homoestra-1,3,5(10),16-tétraén-3-ol
33) 2-propyl-17a-oxo-17a-homo-18a-homoestra-1,3,5(10)-trién-3-ol.

6. D-homoestra-1,3,5(10)-triènes substitués en 2ème position selon l'une quelconque des revendications 1 à 5 ou 2-méthoxy-17a-oxo-17a-homoestra-1,3,5(10)-trién-3-ol pour la préparation d'un médicament.

7. Utilisation de D-homoestra-1,3,5(10)-triènes substitués en 2ème position selon l'une quelconque des revendications 1-5 ou 2-méthoxy-17a-oxo-17a-homoestra-1,3,5(10)-trién-3-ol pour la préparation d'un médicament pour la prophylaxie et la thérapie de maladies estrogéno-dépendantes qui se laissent influencer positivement par l'inhibition de la 17β-hydroxystéroïde-déshydrogénase.

8. Utilisation de D-homoestra-1,3,5(10)-triènes substitués en 2ème position selon la revendication 7, où au moins une autre substance active est utilisée en combinaison pour la préparation d'un médicament.

9. Utilisation de D-homoestra-1,3,5(10)-triènes substitués en 2ème position selon la revendication 8, où l'autre substance active est un antiandrogène, un antigestagène, un inhibiteur d'aromatase ou un antiestrogène.

10. Utilisation de D-homoestra-1,3,5(10)-triènes substitués en 2ème position selon l'une quelconque des revendications 7 à 9 pour la préparation d'un médicament destiné à la prophylaxie et la thérapie de maladies tumorales hormono-dépendantes des glandes génitales mâles et femelles, d'organes sexuels mâles et femelles, y compris les glandes mammaires.

11. Utilisation de D-homoestra-1,3,5(10)-triènes substitués en 2ème position selon la revendication 10 pour la préparation d'un médicament pour la prophylaxie et la thérapie du carcinome mammaire.

12. Utilisation de D-homoestra-1,3,5(10)-triènes substitués en 2ème position selon la revendication 10 pour la préparation d'un médicament destiné à la prophylaxie et la thérapie du carcinome de la prostate.

13. Utilisation de D-homoestra-1,3,5(10)-triènes substitués en 2ème position selon la revendication 10 pour la préparation d'un médicament destiné au traitement de l'endométriose.

14. Compositions pharmaceutiques contenant au moins un composé de formule générale I selon l'une quelconque des revendications 1 à 5 ou du 2-méthoxy-17a-oxo-17a-homoestra-1,3,5(10)-trién-3-ol et le cas échéant au moins une autre substance active ensemble avec des adjuvants et/ou des supports pharmaceutiquement acceptables, l'autre substance active étant un antiandrogène, un antigestagène, un inhibiteur d'aromatase et un antiestrogène.
